# EUROPEAN PATENT SPECIFICATION

(11) **EP 1 465 590 B1**
(45) Date of publication and mention of the grant of the patent: **26.03.2008**
(21) Application number: 02771959.0
(22) Date of filing: 01.11.2002
(51) Int. Cl.: A61K 9/00

(54) **MICELLE COMPOSITIONS CONTAINING PEGYLATED PHOSPHOLIPIDS AND A PHOTOSENSITIZER**
MICELLE-ZUSAMMENSETZUNGEN ENTHALTEND PEGYLIERTEN PHOSPHOLIPIDE UND EINEN PHOTOSENSIBILISATOR
FORMULATIONS CONTENANT UN LIPIDE-POLYETHYLENE GLYCOL

(30) Priority: 02.11.2001 US 337884 P
(43) Date of publication of application: 13.10.2004
(73) Proprietor: The Governors of the University of Alberta, Edmonton, Alberta T6G 2E1 (CA); QLT Inc., Vancouver, British Columbia V5T 4T5 (CA)
(72) Inventor: ALLEN, Theresa, Mary, Edmonton, Alberta T6G 0B4 (CA); BOCH, Ronald, Erwin, North Vancouver,British Columbia V7V 3K2 (CA); BOEY, Anthony, S. K., c/o Northern Lipids, Inc., Vancouver, V6S 2L2 (CA)
(74) Representative: Brasnett, Adrian Hugh
(86) International application number: PCT/CA2002/001690
(87) International publication number: WO 2003/037295

(56) References cited:
- EP-A- 0 569 113
- WO-A-01/08660
- WO-A-01/54666
- WO-A-01/55151
- WO-A-96/23524
- WO-A-02/083097
- US-A- 6 043 237
- SOU K ET AL: "Poly(ethylene glycol)-Modification of the Phospholipid Vesicles by Using the Spontaneous Incorporation of Poly(ethylene glycol)-Lipid into Vesicles" BIOCONJUGATE CHEMISTRY, AMERICAN CHEMICAL SOCIETY, WASHINGTON, US, vol. 11, no. 3, 14 April 2000 (2000-04-14), pages 372-379, XP002209415 ISSN: 1043-1802
- PERKINS W R ET AL: "NOVEL THERAPEUTIC NANO-PARTICLES (LIPOCORES): TRAPPING POORLY WATER SOLUBLE COMPOUNDS" INTERNATIONAL JOURNAL OF PHARMACEUTICS, AMSTERDAM, NL, vol. 200, no. 1, 25 April 2000 (2000-04-25), pages 27-39, XP001159101 ISSN: 0378-5173
- EDWARDS K ET AL: "EFFECT OF POLYETHYLENEGLYCOL-PHOSPHOLIPIDS ON AGGREGATE STRUCTURE IN PREPARATIONS OF SMALL UNILAMELLAR LIPOSOMES" BIOPHYSICAL JOURNAL, NEW YORK, US, US, vol. 73, July 1997 (1997-07), pages 258-266, XP001098663 ISSN: 0006-3495
- ISHIDA T ET AL: "A combinatorial approach to producing sterically stabilized (Stealth) immunoliposomal drugs" FEBS LETTERS, ELSEVIER SCIENCE PUBLISHERS, AMSTERDAM, NL, vol. 460, no. 1, 22 October 1999 (1999-10-22), pages 129-133, XP004342732 ISSN: 0014-5793
- JOHNSSON M ET AL: "PHASE BEHAVIOR AND AGGREGATE STRUCTURE IN MIXTURES OF DIOLEOYLPHOSPHATIDYLETHANOLAMINE AND POLY(ETHYLENE GLYCOL)-LIPIDS" BIOPHYSICAL JOURNAL, vol. 80, January 2001 (2001-01), pages 313-323, XP002274832

## Description

The invention is generally related to the use of polyethylene glycol (PEG) and lipid containing amphipathic molecules in micelles and their use in the delivery of chemically and biologically active agents. The micelles of the invention are particular useful for the rapid release of such agents. An example of an agent that may be delivered by the micelles of the invention is a photosensitizer useful in pharmaceutical, agricultural, or industrial applications. The invention also relates to processes for the production of, and application of, said micelles as a delivery system for one or more active agents.

### BACKGROUND OF THE INVENTION

While many active agents are hydrophobic or otherwise water insoluble, they are often needed in water based or otherwise aqueous environments. As such, multiple systems have been developed as delivery vehicles for such agents. These include the use of organic solvents, aqueous/detergent mixtures, aqueous/organic solvent mixtures (such as co-solvents), emulsions, liposomes, and micelles. For example, Parikh et al., U.S. Pat. 5,922,355, disclose microparticles comprising insoluble substances.

Liposome systems have also been improved. Liposome systems, for example, have been modified to enhance their stability and circulation time (see for example U.S. Patents 4,837,028 and 4,920,016) as well as their ability to target particular cells or tissues (see for example U.S. Patents 5,527,528 and 5,620,689).

Micelles have been used to deliver medications to patients, (Brodin et al., Acta Pharm. Suec. 19 267-284 (1982)) and micelles have been used as drug carriers and for targeted drug delivery, (Supersaxo et al., Pharm. Res. 8:1286-1291 (1991)), including cancer medications, (Fung et al., Biomater. Artif. Cells. Artif. Organs 16: 439 et. seq. (1988); and Yokoyama et al., Cancer Res. 51: 3229-3236 (1991)). Lasic (Nature, Vol. 355, pp. 379-380, (1992)) describes the use of mixed micelles comprising a drug agent and biological lipids.

Amphipathic molecules comprising lipid and hydrophilic (such as polyethylene glycol (PEG)) portions are surfactants that have a tendency to spontaneously form colloidal aggregates in aqueous solution, known as micelles, when monomer content is above a certain critical micelle concentration (CMC). See for example Kwun et al. "Polymeric micelles as new drug carriers" Adv. Drug Del. Rev. 21:107-116 (1996); and Bedu-Addo, et al. "Effects of polyethyleneglycol chain length and phospholipid acyl chain composition on the interaction of polyethyleneglycol-phospholipid conjugates with phospholipid: implications in liposomal drug delivery" Pharm Res. 13:710-717 (1996).

Micelles have been of great interest as slow release, long circulation drug delivery vehicles. See for example Yokoyama et al. "Toxicity and antitumor activity against solid tumors of micelle-forming polymeric anticancer drug and its extremely long circulation in blood" Cancer Res. 51:3229-3236 (1991); and Trubetskoy et al. "Use of polyethylene-lipid conjugate as long circulating carriers for delivery of therapeutic and diagnostic agents" Adv. Drug Del. Rev. 16:311-320 (1995).

There are situations, however, where a more rapid release of a hydrophobic agent is preferred. One example is conventional photodynamic therapy (PDT), which generally involves the administration of a photosensitizer drug or compound to a recipient, either locally or systemically, followed by irradiation with light that is capable of being absorbed by the photosensitizer in the tissue or organ to be treated. While some photosensitizers, such as Photofrin® (Axcan Pharmaceuticals, Canada) may be delivered as part of a simple aqueous solution, other hydrophobic photosensitizers have a tendency to aggregate in aqueous solutions by molecular stacking, which can severely curtail subsequent photosensitization processes (Siggel et al. J. Phys. Chem. 100(12):2070-2075, Dec 1996).

Hydrophobic photosensitizers of great interest include the polypyrrolic macrocycle based compounds and, in particular green porphyrins such as BPD-MA (benzoporphyrin derivative monoacid ring A). These compounds have been known for some time to be useful, when combined with light, for the treatment and diagnosis of a variety of conditions, including tumors, angiogenesis and neovasculature, restenosis and atherosclerotic plaques, and rheumatoid arthritis. The porphyrins have a natural tendency to "localize" in malignant or proliferating tissue, where they absorb light at certain wavelengths when irradiated. The absorbed light may result in a cytotoxic effect in the cells, and neighboring cells, into which the porphyrins have localized. (See, e.g., Diamond et al., Lancet, 2:1175-77 (1972); Dougherty et al., "The Science of Photo Medicine", 625-38 (Regan et al. eds. 1982); and Dougherty et al., "Cancer: Principles and Practice of Oncology", 1836-44 (DeVita Jr. et al. eds. 1982)). It has been postulated that the cytotoxic effect of porphyrins is due to the formation of singlet oxygen when exposed to light (Weishaupt et al., Cancer Research, 36:2326-29 (1976)).

Of particular interest is a group of modified porphyrins, known as "green porphyrins" (Gp), having one or more light absorption maxima between about 670-780 nm. These Gp compounds, used in conjunction with light, have been shown to confer cytotoxicity against target cells at concentrations lower than those required for hematoporphyrin or HPD. Gp compounds can be obtained using Diels-Alder reactions of protoporphyrin with various acetylene derivatives under the appropriate conditions. Preferred forms of Gp are the hydro-monobenzoporphyrin derivatives ("BPD's") as well as BPD-MA (including the compound known by the generic name verteporfm), EA6 (including the compound known as QLT 0074) and B3 in particular. The preparation and use of the Gp and BPD compounds are disclosed in U.S. Patent Nos. 4,920,143, 4,883,790 and 5,095,030, hereby incorporated by reference into the disclosure of the present application. The preparation and uses of EA6 and B3 are disclosed in U.S. Patent Nos. 6,153,639 and 5,990,149 respectively, also hereby incorporated by reference.

Many desirable hydro-monobenzoporphyrin photosensitizers, such as BPD-MA, are not only insoluble in water at physiological pH's, but are also insoluble in pharmaceutically acceptable aqueous-organic co-solvents. Thus liposomal formulations of BPD-MA (verteporfin) and zinc phthalocyanine (CIBA-Geigy Ltd., Basel, Switzerland) have been used. The liposome in the case of BPD-MA acts as a passive delivery agent, transferring the photosensitizer to plasma lipoproteins, such as low density lipoproteins (LDL), immediately upon injection into the blood stream. The higher surface expression of LDL receptors in rapidly proliferating tissues affords a level of selectivity to localization of hydrophobic LDL associated drugs at target sites for PDT.

Similarly, hematoporphyrin (HP) and hematoporphyrin dimethyl esters have been formulated in unilamellar vesicles of dipalmitoyl phosphatidyl choline (DPPC) and liposomes of dimyristoyl (DMPC) and distearoyl phosphatidyl choline (DSPC). Zhou et al., supra; Ricchelli, New Directions in Photodynamic Therapy, 847:101-106 (1987); Milanesi, Int. J. Radiat. Biol., 55:59-69 (1989). HP, porfimer sodium, and tetrabenzoporphyrins have been formulated in liposomes composed of egg phosphatidyl choline (EPC). Johnson et al., Proc. Photodynamic Therapy: Mechanisms II, Proc. SPIE-Int. Soc. Opt. Eng., 1203:266-80 (1990).

Further, freeze-dried pharmaceutical formulations comprising a porphyrin photosensitizer, a disaccharide or polysaccharide, and one or more phospholipids (such as EPG and DMPC) have been made. These formulations form liposomes containing an effective amount of porphyrin photosensitizer upon reconstitution with a suitable aqueous vehicle and are described in Desai et al., U.S. Patent No. 6,074,666, which is incorporated by reference. Methods for the large-scale production of DMPC/EPG liposomes containing a photosensitizer are disclosed in U.S. Pat. 5,707,608, which is incorporated by reference as if fully set forth.

In PDT, a rapid release of the photosensitizer (PS) from the delivery system is often preferred to permit administration of an effective dose of activating light within a conveniently short period of time after PS administration. Rapid release also permits the PS to begin clearance from the subject to minimize spurious activation by ambient light after administration of activating light.

Additionally, a PS delivery system for PDT is preferably simple, non-toxic (biodegradable or readily excreted), chemically inert, economical and easily used while maintaining the drug in a relatively non-aggregated form with an extended shelf life (preferably as a solid state formulation). The actual delivery vehicle should be effective in delivering the photosensitizer, easy to reconstitute for use, and suitable for sterilization by filtration in the event that autoclaving or gamma-radiation is not suitable.

### SUMMARY OF THE INVENTION

The present invention provides micelle compositions comprising polyethylene glycol (PEG) covalently conjugated to phospholipids as well as methods for their preparation and use. While the compositions may serve as vehicles to contain or deliver any chemically or biologically active agent, they are preferred as vehicles for photosensitizers. In contrast to some PEG micelle systems described in the prior art, it has been discovered that the micelle compositions of the invention are able to release an active agent *in vivo* relatively quickly, depending on the photosensitizer chosen, and thus have the potential to address many needs and formulation requirements of photosensitizer delivery systems. It has also been discovered that the compositions of the invention maintain hydrophobic agents in a non-aggregated form and at a relatively high concentration while at a low (total) lipid to active agent ratio. The lipids of the micelles may, of course, include other lipids or phospholipids in addition to a PEG containing phospholipid.

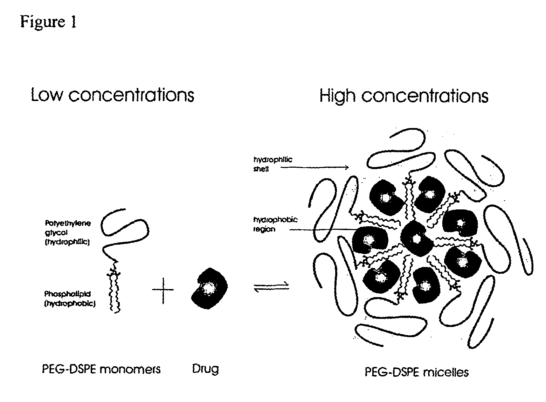

The invention further provides methods of preparing the aforementioned compositions. These methods comprise combining an active agent, such as a photosensitizer (PS), and one or more PEG-containing phospholipids , which are capable of forming micelles, followed by conversion into a solid form, if so desired. The solid form compositions containing the active agent (such as a PS) and PEG-containing phospholipids may remain as a solid, or be hydrated with an aqueous solution without loss of the micelle physical properties, for storage or application. The solid form compositions may be formulated to comprise one or more hydration enhancing compounds, which make hydration of the micelles simpler, quicker, and/or more efficient. A schematic representation of the micelles of the invention is shown in Figure 1.

The compositions, either before or after hydration, may be further combined with other pharmaceutically acceptable agents. The solid or hydrated form of the composition may be separated into doses appropriate for administering an effective amount of the photosensitizer to a subject.

The present invention also provides methods for administration of the micelles to subjects in need of particular active agents. In the case of photosensitizers, the micelles are administered to subjects undergoing photodynamic therapy.

### BRIEF DESCRIPTION OF THE DRAWINGS

Figure 1 is a schematic representation of the reversible interaction of a hydrophobic drug with polyethylene glycol-lipid conjugate micelles.
Figure 2 is a graph showing the measurement of the critical micelle concentration (CMC) of P2K-DSPE before and after incorporation of QLT0069.

### DETAILED DESCRIPTION OF THE INVENTION

### DEFINITIONS

Prior to setting forth the invention, it may be helpful to an understanding thereof to first set forth definitions of certain terms that will be used hereinafter.

"Amphipathic" or "amphipathic molecule" refers to the presence of both a hydrophobic and hydrophilic moiety in a single molecule. The hydrophobic moiety may be lipophilic, and the hydrophilic moiety may be polar and/or charged. Hydrophobic refers to any substance or portion thereof which is more soluble in a nonpolar solvent than in a polar solvent. Hydrophilic refers to any substance or portion thereof which is more soluble in a polar solvent than in a non-polar solvent. Preferred amphipathic molecules of the invention are those which are capable of self assembly into micelles. A preferred hydrophilic portion for the practice of the invention comprises polyethylene glycol (PEG).

"Micelle" refers to a colloidal aggregate formed from amphipathic molecules at a concentration above a critical micelle concentration (CMC). Micelles are distinguished from liposomes in that the liposomes comprise one or more lipid bilayers while micelles do not. Moreover, the hydrophobic (lipophilic) "tail" portion of the phospholipids generally oriented toward the interior of the micelle. Preferably, micelles have the "tail" portion generally oriented toward the center of the micelle. Micelles do not have a bilayer structure and so are not considered vesicles or liposomes. Micelles may also be formed in a reverse orientation wherein the hydrophobic portions of the amphipathic molecules face the exterior of the micelle while the hydrophilic portions of the molecules face the interior of the micelle. Micelles of the invention are preferably small (less than 200 nanometers (nm)) and contain high concentrations of an active agent, such as near or about 2 mg/mL, in a low (molar) lipid:active agent ratio. More preferred are micelles with average diameters of less than about 30 nm. Even more preferably, they have average diameters of less than about 20 nm. Micelles of the invention preferably contain concentrations of a photosensitizer like QLT 0069 of about 2 mg/mL.

"Lipid" refers to a hydrophobic substance. Preferably, they are fatty acids containing at least 10 carbon atoms, more preferably about 12, about 14, about 16, about 18, about 20, about 22, or about 24 carbon atoms. Fatty acid chains of more than 24 carbon atoms, as well as other hydrophobic substances, such as, but not limited to, cholesterol may be used. The fatty acid chains may be fully or partially saturated. Particular fatty acid chains have names (followed by the number of carbon atoms they possess) such as laurate (12C), myristate (14C), palmitate or palmitoleate (16C), stearate or oleate or linoleate or linolenate (18C), arachidate or arachidonate (20C), or behenate (22C), and lignocerate (24C).

"Phospholipid" refers to amphipathic molecules comprising a lipid portion and a phosphorus-containing hydrophilic portion. In molecules comprising glycerol, the hydrophilic portion is preferably phosphatidylserine, phosphatidylethanolamine, phosphatidylcholine, or phosphatidylinositol. In particularly preferred embodiments of the invention, the phospholipid is a distearoylphosphatidylethanolamine (DSPE).

As used herein, polyethylene glycol or PEG, refers to such compounds having a molecular weight between about 100 to 10,000 daltons depending on the number of ethylene oxide units in the polymer chain. Preferred molecular weights (MW) are from about 500 to about 10,000, about 1000 to 10,000 (or about 22 to 220 ethylene oxide units), about 2000 to 10,000, and about 3000 or 4000 to 10,000. Particularly preferred embodiments are PEG having a molecular weight about 2000, although molecular weights of about 5000, about 6000, about 7000, and about 8000 may also be used in the practice of the invention. In particularly preferred embodiments of the invention, PEG of 2000 MW are used with DSPE.

"Green porphyrins" refer to porphyrin derivatives obtained by reacting a porphyrin nucleus with an alkyne in a Diels-Alder type reaction to obtain a mono-hydrobenzoporphyrin.

In addition to the micelle and micelle-containing compositions of the invention, the present invention provides methods for formulating said micelles. In one embodiment, such methods involve dissolving the amphipathic molecule, such as PEG₂₀₀₀-DSPE, and one or more active agent in a suitable solvent, such as dichloromethane, followed by solvent removal to form a thin film. The thin film may be hydrated with an aqueous solvent to form a solution comprising micelles for administration or application or for sterilization by a 0.22 µm filter. The film may also be divided into portions before being individually hydrated. Alternatively, the micelles may be formed by adding a miscible volatile solvent containing a PS and PEG-lipid to an aqueous phase, such that the organic phase is removed (e.g. by heating the mixture), leaving the aqueous micelle-containing PS in solution. Various amounts of active agent may be used within suitable ranges of the (molar) lipid:active agent ratio. Preferred ratios are from about 0.5 to about 10 or about 20. Ratios of about 0.5, about 1, about 2, about 3, about 4, about 5, about 6, about 7, about 8, and about 9, about 10, up to about 19 may also be used. Preferred ratios for the practice of the invention are from about 2 to about 6 or about 6 to about 10. Additionally within the scope of the invention are the intermediate ratios within the range, such as from about 4.1:1 to 4.9:1, about 5.1:1 to 5.9:1, about 6.1:1 to 6.9:1, about 7.1:1 to 7.9:1, and about 8.1:1 1 to 8.9:1, are within the scope of the invention.

Hydration may be with any suitable aqueous solution, including buffered or non-buffered solutions (such as distilled water or water for injection). When buffered solutions are used, they are preferably buffered at a pH of about 5 to about 9, more preferably at a pH of about 5.5, about 6.0, about 6.5, about 7.0, about 7.5, about 8.0, and about 8.5. Examples of buffered solutions include, but are not limited to, 2 or 20 mM phosphate buffered solutions.

The compositions and methods of the present invention further include administration of active agent-containing micelles as delivery vehicles to a subject in need of the agent. Preferably, the active agent is not a polypeptide molecule or not a polypeptide comprising more than about 15 or about 25 amino acids. Instead, the active agent is preferably a small organic molecule with a molecular weight greater than 600 Daltons. For example, micelles of the invention may be used to deliver photosensitizer compounds for recipients undergoing PDT treatment.

### Photosensitizers

The invention may be practiced with a variety of synthetic and naturally occurring pyrrole based photosensitizers, including pro-drugs such as 5-aminolevulinic acid, porphyrins and porphyrin derivatives e.g. chlorins, bacteriochlorins, isobacteriochlorins, phthalocyanine and naphthalocyanines and other tetra- and poly-macrocyclic compounds, and related compounds (e.g. pyropheophorbides, sapphyrins and texaphyrins) and metal complexes (such as, but not limited by, tin, aluminum, zinc, lutetium). Tetrahydrochlorins, purpurins, porphycenes, and phenothiaziniums are also within the scope of the invention.

Particularly preferred photosensitizers include green porphyrins such as BPD-MA, EA6 and B3. Generally, any polypyrrolic or tetrapyrrolic macrocyclic photosensitive compound that is hydrophobic can be used in the invention. Examples of these and other photosensitizers for use in the present invention include, but are not limited to, angelicins, some biological macromolecules such as lipofuscin; photosystem II reaction centers; and D1-D2-cyt *b*-559 photosystem II reaction centers, chalcogenapyrillium dyes, chlorins, chlorophylls, coumarins, cyanines, ceratin DNA and related compounds, certain drugs such as adriamycin; afloqualone; amodiaquine; daunomycin; daunomycinone, certain flavins riboflavins, fullerenes, metalloporphyrins, metallophthalocyanines, methylene blue derivatives, naphthalimides, naphthalocyanines, certain natural compounds such as kynurenines; sanguinarine; berberine; carmane; and 5,7,9(11),22-ergostatetraene-3 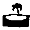 -ol, nile blue derivatives, NSAIDs (nonsteroidal anti-inflammatory drugs), perylenequinones, phenols, pheophorbides, pheophytins, photosensitizer dimers and conjugates, phthalocyanines, porphycenes, porphyrins, psoralens, purpurins, quinones, retinoids, rhodamines, thiophenes, verdins, vitamins and xanthene dyes (Redmond and Gamlin, Photochem. Photobiol., 70(4):391-475 (1999)).

Exemplary angelicins include those modified by aceto or methyl groups at the 3, 4', 4, 5', and/or 6 positions.

Exemplary chalcogenapyrillium dyes include pyrilium, selenopyrilium, thiopyrilium and telluropyrilium perchlorates.

Exemplary chlorins dyes include 5-azachlorin dimethyl ester derivative; 5,10,15,20-tetrakis-(*m*-hydroxyphenyl) bacteriochlorin; benzoporphyrin derivative monoacid ring A; benzoporphyrin derivative monoacid ring-A; porphine-2,18-dipropanoic acid, 7-[2-dimethyl-amino)-2-oxoethyl]-8-ethylidene-7,8-dihydro-3,7,12,17-tetramethyl, dimethylester; porphine-2,18-dipropanoic acid, 7-[2-dimethyl-amino)-2-oxoethyl]-8-ethylidene-8-ethyl-7,8-dihydro-3,7,12,17-tetramethyl, dimethylester Z; porphine-2,18-dipropanoic acid, 7-[2-dimethyl-amino)-2-oxoethyl]-8-ethylidene-8-ethyl-7,8-dihydro-3,7,12,17-tetramethyl, dimethylester Z ECHL; porphine-2,18-dipropanoic acid, 7-[2-dimethyl-amino)-2-oxoethyl]-8-ethylidene-8-*n*-heptyl-7,8-dihydro-3,7,12,17-tetramethyl, dimethylester Z; tin (II) porphine-2,18-dipropanoic acid, 7-[2-(dimethylamino-2-oxoethyl]-8-ethylidene-8-*n*-heptyl-7,8-dihydro-3,7,12,17-tetramethyl, dimethylester Z; chlorin *e*₆; chlorin *e*₆ dimethyl ester; chlorin *e*₆ k₃; chlorin *e*₆ monomethyl ester; chlorin *e*₆ Na₃; chlorin *p*₆; chlorin *p*₆-trimethylester; chlorin derivative zinc (II) porphine-2,18-dipropanoic acid, 7-[2-(dimethylamino)-2-oxoethyl]-8-ethylidene-8-*n*-heptyl-7,8-dihydro-3,7,12,17-tetramethyl, dimethylester Z; 13¹-deoxy-20-formyl-*vic*-dihydroxy-bacteriochlorin di-*tert*-butyl aspartate; 13'-deoxy-20-formyl-4-keto-bacteriochlorin di-*tert*-butyl aspartate; di-L-aspartyl chlorin *e*₆; mesochlorin; 5,10,15,20-tetrakis-(*m*-hydroxyphenyl) chlorin; *meta*-(tetrahydroxyphenyl)chlorin; methyl-13¹-deoxy-20-formyl-4-keto-bacteriochlorin; mono-L-aspartyl chlorin *e*₆; photoprotoporphyrin IX dimethyl ester; phycocyanobilin dimethyl ester; protochlorophyllide *a*; tin (IV) chlorin *e*₆; tin chlorin *e*₆; tin L-aspartyl chlorin *e*₆; tin octaethyl-benzochlorin; tin (IV) chlorin; zinc chlorin *e*₆; and zinc L-aspartyl chlorin *e*₆.

Exemplary chlorophylls dyes include chlorophylls *a* and *b*; bacteriochlorophylls *a, b, c,* or d; protochlorophylls; and amphiphilic derivatives thereof.

Exemplary coumarins include methoxycoumarins; thenoylcoumarins; khellin; RG 708; RG277; and visnagin.

Exemplary cyanines include benzoselenazole dye; benzoxazole dye; oxacarbocyanines; thiacarbocyanines; selenacarbocyanines; kryptocyanine; benzoxazole derivatives; quinoline derivatives; and merocyanines.

Exemplary fullerenes include C₆₀; C₇₀; C₇₆; dihydro-fullerenes; buckminster-fullerenes; and tetrahydro fullerenes.

Exemplary metalloporphyrins include chlorotexaphyrin nitrates; cadmium or cobalt or copper or Europium or gallium or lutetium or magnesium or manganese or nickel or palladium or platinum or samarium or silver or tin or zinc porphyrins, tetrabenzoporphyrins, porphines, texaphyrins, hematoporphyrins, tetrabenzoporphyrins, tetraphenylporphyrins, chlorotexaphyrins, porphyrazines; zinc protoporphyrin; and zinc protoporphyrin IX.

Exemplary metallophthalocyanines include aluminum chloroaluminum cobalt or copper or dichlorosilicon or gallium or germanium or lead or magnesium or nickel or palladium or ruthenium or silicon or tin or vanadium phthalocyanines (optionally sulfonates, disulfonates, trisulfonates, and tetrasulfonates).

Exemplary naphthalimides blue derivatives include *N*,*N'*-bis-(hydroperoxy-2-methoxyethyl)-1,4,5,8-naphthaldiimide; *N*-(hydroperoxy-2-methoxyethyl)-1,8-naphthalimide; 1,8-naphthalimide; *N*,*N'*-bis(2,2-dimethoxyethyl)-1,4,5,8-naphthaldiimide; and *N*,*N'*-bis(2,2-dimethylpropyl)-1,4,5,8-naphthaldiimide.

Exemplary naphthalocyanines include aluminum or silicon or zinc Naphthalocyanines, chloronaphthalocyanines, *t*-butylnaphthalocyanines, amidonaphthalocyanines, tetraaminonaphthalocyanines, tetrabenzamidonaphthalocyanines, tetrahexylamidonaphthalocyanines, tetrarnethoxy-benzarnidonaphthalocyanines, tetramethoxynaphthalocyanines, naphthalocyanine tetrasulfonates and tetradodecylamidonaphthalocyanines.

Exemplary nile blue derivatives include benzo[a]phenothiaziniums.

Exemplary perylenequinones include hypericins, calphostin C, cercosporins, elsinochromes, phleichromes and rubellin A.

Exemplary phenols include 2-benzylphenol; 2,2'-dihydroxybiphenyl; 2,5-dihydroxybiphenyl; 2-hydroxybiphenyl; 2-methoxybiphenyl; and 4-hydroxybiphenyl.

Exemplary pheophorbides include pheophorbide *a*; methyl 13¹-deoxy-20-formyl-7,8-*vic*-dihydro-bacterio-*meso*-pheophorbide *a*; methyl-2-(1-dodecyloxyethyl)-2-devinyl-pyropheophorbide *a*; methyl-2-(1-heptyl-oxyethyl)-2-devinyl-pyropheophorbide *a*; methyl-2-(1-hexyl-oxyethyl)-2-devinyl-pyropheophorbide *a*; methyl-2-(1-methoxy-ethyl)-2-devinyl-pyropheophorbide *a*; methyl-2-(1-pentyl-oxyethyl)-2-devinyl-pyropheophorbide *a*; magnesium methyl bacteriopheophorbide *d*; methyl-bacteriopheophorbide *d*; and pheophorbide.

Exemplary pheophytins include bacteriopheophytin *a*; bacteriopheophytin *b*; bacteriopheophytin *c*; bacteriopheophytin *d*; 10-hydroxy pheophytin *a*; pheophytin; pheophytin *a*; and protopheophytin.

Exemplary porphyrins include 5-azaprotoporphyrin dimethylester; *bis*-porphyrin; coproporphyrin III; coproporphyrin III tetramethylester; deuteroporphyrin; deuteroporphyrin IX dimethylester; diformyldeuteroporphyrin IX dimethylester; dodecaphenylporphyrin; hematoporphyrin; hematoporphyrin IX; hematoporphyrin monomer; hematoporphyrin dimer; hematoporphyrin derivatives; hematoporphyrin IX dihydrochloride; hematoporphyrin IX dimethylester; mesoporphyrin dimethylester; monoformyl-monovinyl-deuteroporphyrin IX dimethylester; monohydroxyethylvinyl deuteroporphyrin; 5,10,15,20-tetra(o-hydroxyphenyl) porphyrin; 5,10,15,20-tetra(*m*-hydroxyphenyl) porphyrin; 5,10,15,20-tetrakis-(*m*-hydroxyphenyl) porphyrin; 5,10,15,20-tetra(*p*-hydroxyphenyl) porphyrin; 5,10,15,20-tetrakis (3-methoxyphenyl) porphyrin; 5,10,15,20-tetrakis (3,4-dimethoxyphenyl) porphyrin; 5,10,15,20-tetrakis (3,5-dimethoxyphenyl) porphyrin; 5,10,15,20-tetrakis (3,4,5-trimethoxyphenyl) porphyrin; 2,3,7,8,12,13,17,18-octaethyl-5,10,15,20-tetraphenylporphyrin; Photofrin®; Photofrin® II; porphyrin c; protoporphyrin; protoporphyrin IX; protoporphyrin dimethylester; protoporphyrin IX dimethylester; protoporphyrin propylaminoethylformamide iodide; protoporphyrin *N*,*N*-dimethylaminopropylformamide; protoporphyrin propylaminopropylformamide iodide; protoporphyrin butylformamide; protoporphyrin *N*,*N-*dimethylamino-formamide; protoporphyrin formamide; sapphyrins; porphines; tetrakis(4-sulfonatophenyl)porphyrin; *meso*-tetra(4-N-trimethylanilinium)-porphine; uroporphyrin; uroporphyrin IX; and uroporphyrin I.

Exemplary psoralens include methoxypsoralens dimethoxypsoralens; carbethoxypsoralens; pseudopsoralens; hydroxypsoralens; trimethylpsoralens; allopsoralens; isopseudopsoralen; acetoisopseudopsoralens; pseudoisopsoralens; and acetopseudoisopsoralens.

Exemplary purpurins include octaethylpurpurin; octaethylpurpurin zinc; oxidized octaethylpurpurin; reduced octaethylpurpurin; reduced octaethylpurpurin tin; purpurin 18; purpurin-18; purpurin-18-methyl ester; purpurin; tin ethyl etiopurpurin I; Zn(II) aetio-purpurin ethyl ester; and zinc etiopurpurin.

Exemplary quinones include anthraquinones; benzoquinones; hydroquinones; chlorohydroquinones; resorcinol; and 4-chlororesorcinol.

Exemplary retinoids include all-*trans* retinal; C₁₇ aldehyde; C₂₂ aldehyde; 11-*cis* retinal; 13-*cis* retinal; retinal; and retinal palmitate.

Exemplary thiophenes include terthiophenes, bithiophenes, diphenylthiophene; quaterthiophenes; 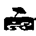 -quaterthienyl; 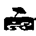 -tetrathiophene; 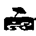 -pentathiophene; 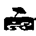 -hexathiophene; and 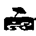 -heptathiophene.

Exemplary verdins include copro (II) verdin trimethyl ester; deuteroverdin methyl ester; mesoverdin methyl ester; and zinc methyl pyroverdin.

Exemplary vitamins include ergosterol (provitamin D2); hexamethyl-Co *a* Co *b-*dicyano-7-de(carboxymethyl)-7,8-didehydro-cobyrinate (Pyrocobester); pyrocobester; and vitamin D3.

Exemplary xanthene dyes include eosins and eosin derivatives, erythrosins, fluoresceins, phloxins, and rose bengals.

In one embodiment the preferred compounds for formulating are the highly hydrophobic tetrapyrrolic A and B-ring compounds, such as BPD-DA, -DB, -MA, and -MB. Most preferred are the B-ring compounds, BPD-MB, B-EA6, B-B3; the A-ring compounds BPD-MA, A-EA6 and A-B3; and dihydroxychlorins.

These compounds are porphyrin derivatives obtained by reacting a porphyrin nucleus with an alkyne in a Diels-Alder type reaction to obtain a monohydrobenzoporphyrin, and they are described in detail in the issued U.S. Pat. No. 5,171,749, which is hereby incorporated in its entirety by reference. Of course, combinations of photosensitizers may also be used. It is preferred that the absorption spectrum of the photosensitizer be in the visible range, typically between 350 nm and 1200 nm, more preferably between 400-900 nm, and even more preferably between 600-900 nm.

BPD-MA is described, for example, in U.S. Patent Nos. 5,171,749 and 5,095,030; EA6 and B3 are described in U.S. Patent Nos. 5,929,105 and5,880,145, respectively, all of which are incorporated herein by reference. Preferred green porphyrins have the basic structure: 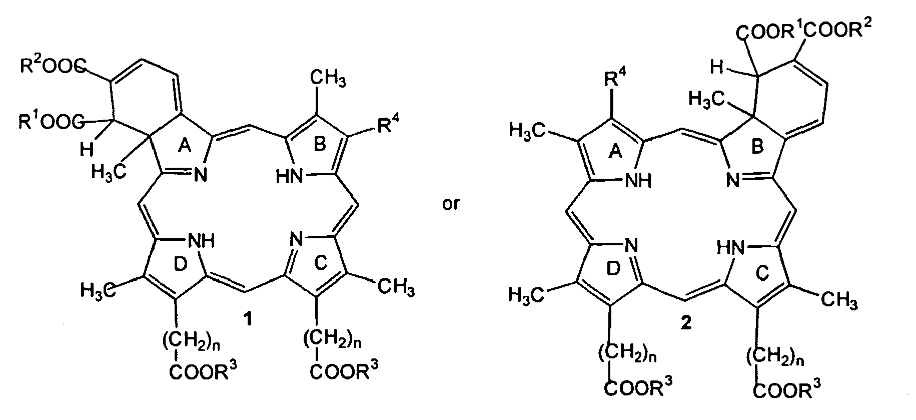 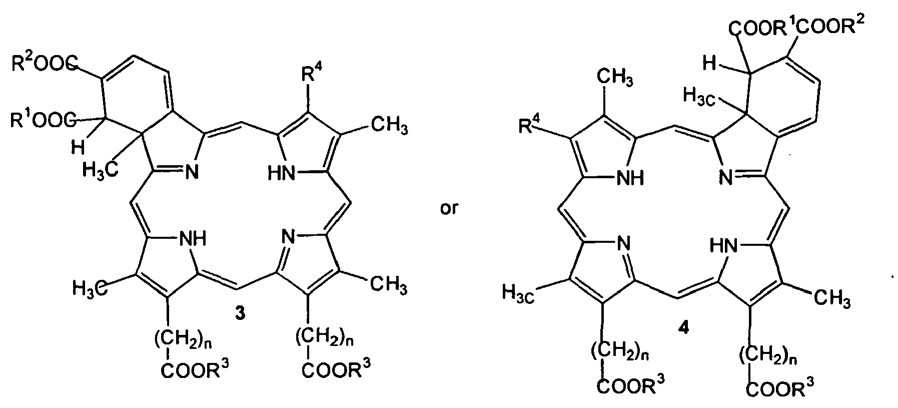 where R⁴ is vinyl or 1-hydroxyethyl and R¹, R², and R³ are H or alkyl or substituted alkyl, and n is an integer between 0 and 6, preferably 2.
BPD-MA (verteporfin) has the structure shown in formula 1 wherein R¹ and R² are methyl, R⁴ is vinyl and one of R³ is H and the other is methyl, and n=2. B-EA6 is of formula 2 wherein R¹ and R² are methyl and both R³ are 2-hydroxyethyl (i.e., the ethylene glycol esters). B3 is of formula 2 wherein R¹ is methyl, R² is H, and both R³ are methyl, and n=2. In both EA6 and B3, R⁴ is also vinyl.

The representations of BPD-MA_{C} and BPD-MA_{D}, which are the enantiomeric components of verteporfin, as well as illustrations of A and B ring forms of EA6 and B3 (where n=2), are as follows:

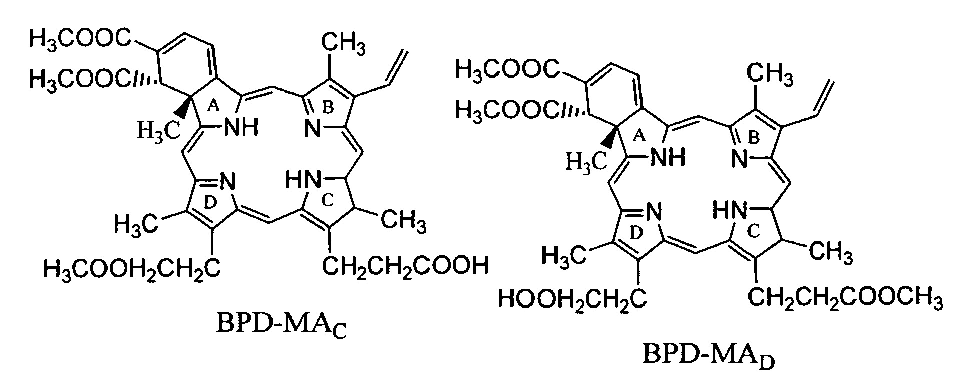

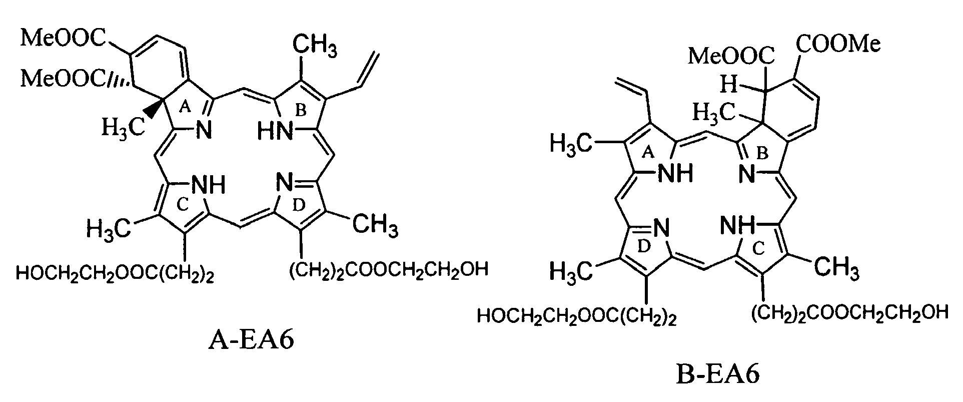

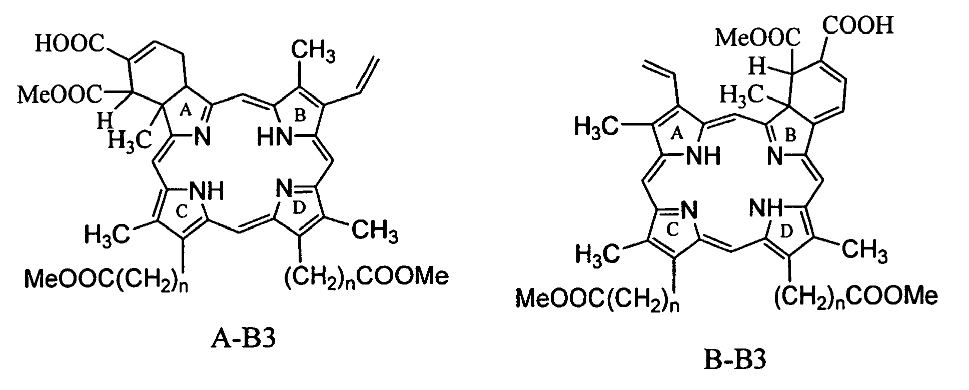

Related compounds of formulas 3 and 4 are also useful; in general, R⁴ will be vinyl or 1-hydroxyethyl and R¹, R², and R³ are H or alkyl or substituted alkyl.

Additional examples of hydrophobic BPD B-ring compounds that are difficult to formulate, and are especially well suited to use in the invention are shown below. The compound QLT0069 is used in several of the Examples herein.

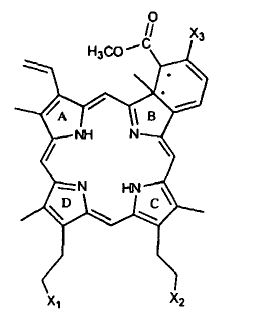

| **Drug** | *X1* | **X2** | **X3** |
|---|---|---|---|
| QLT0060 | CO(O(CH₂)₂)0H | CO(O(CH₂)₂)0H | COOCH₃ |
| QLT0069 | COOCH₃ | COOCH₃ | COOH |
| QLT0078 | CO(O(CH₂)₂)₂0H | CO(O(CH₂)₂)₂0H | COOCH₃ |
| QLT0080 | CO(O(CH₂)₂)₃OH | CO(O(CH₂)₂)₃OH | COOCH₃ |
| QLT0081 | CO(O(CH₂)₂)₂OCH₃ | CO(O(CH₂)₂)₂OCH₃ | CO(O(CH₂)₂)₂OCH₃ |
| QLT0082 | CO(O(CH₂)₂)₂OH | CO(O(CH₂)₂)₂OH | CO(O(CH₂)₂)₂OH |
| QLT0083 | CO(O(CH₂)₂)₃OH | CO(O(CH₂)₂)₃OH | CO(O(CH₂)₂)₃OH |
| QLT0087 | CO(O(CH₂)₂)₄OH | CO(O(CH₂)₂)₄OH | COOCH₃ |
| QLT0088 | COOCH₃ | COOCH₃ | CONH(C₆H₄)(C₅H₁₀N) |
| QLT0090 | CO(O(CH₂)₂)₅OH | CO(O(CH₂)₂)₅OH | COOCH₃ |
| QLT0093 | CO(O(CH₂)₂)₅OH | CO(O(CH₂)₂)₅OH | CO(O(CH₂)₂)₅OH |

Dimeric forms of the green porphyrin and dimeric or multimeric forms of green porphyrin/porphyrin combinations may also be used. The dimers and oligomeric compounds of the invention can be prepared using reactions analogous to those for dimerization and oligomerization of porphyrins per se. The green porphyrins or green porphyrin/porphyrin linkages can be made directly, or porphyrins may be coupled, followed by a Diels-Alder reaction of either or both terminal porphyrins to convert them to the corresponding green porphyrins.

Other non-limiting examples of photosensitizers which may be useful in the invention are photosensitizing Diels-Alder porphyrin derivatives, described in US Patent 5,308,608; porphyrin-like compounds, described in US Patents 5,405,957, 5,512,675, and 5,726,304; bacteriochlorophyll-A derivatives described in US Patents 5,171,741 and 5,173,504; chlorins, isobacteriochlorins and bacteriochlorins, as described in US Patent 5,831,088; meso-monoiodo-substituted and meso substituted tripyrrane, described in US Patent 5,831,088; polypyrrolic macrocycles from meso-substituted tripyrrane compounds, described in US Patents 5,703,230, 5,883,246, and 5,919,923; and ethylene glycol esters, described in US Patent 5,929,105. All of the patents cited in this paragraph are hereby incorporated by reference as if fully set forth. Generally any hydrophobic photosensitizers, which absorb in the ultra-violet, visible and infra-red spectroscopic ranges would be useful for practicing this invention.

Presently a number of photosensitizer drugs of interest are hydrophobic with a tetrapyrrole-based structure. These drugs have an inherent tendency to aggregate, which can severely curtail photosensitization processes (Siggel et al. J. Phys. Chem. 100(12):2070-2075, Dec 1996). For example, the synthetic pathway for BPD yields A and B ring intermediates in approximately equimolar quantities, which can be derivatized further. It was found that the A-ring derivatives, such as BPD-MA (verteporfin), could be formulated for delivery using traditional means such is liposomes, whereas B-ring compounds proved more difficult to formulate due to their tendency to undergo self-association. This self-association of B-ring derivative BPDs into dimers is described in D. Delmarre et al, Can J. Chem. 79: 1069-1074 (2001), which is incorporated by reference herein as if fully set forth. Whereas monomeric B-ring BPDs exhibit a major absorption band at 692 nm, the dimer absorbs at 724 nm. Thus the tendency of B-ring BPDs to undergo self-association in a particular formulation can be assessed by the A692/A720 ratio. The red shift observed in the absorption spectra is most likely a consequence of dimer formation consisting of two head-to-tail stacked molecules of the B-ring BPD.

In an additional aspect of the invention, the photosensitizers of the invention may be conjugated to various ligands that facilitate targeting to tissues and cells before the photosensitizers are formulated with amphipathic molecules. These ligands include those that are receptor-specific as well as immunoglobulins and fragments thereof. Preferred ligands include antibodies in general and monoclonal antibodies, as well as immunologically reactive fragments thereof.

### Micelles

The micelles of the invention contain one or more PEG conjugated phospholipids. PEG-conjugated phospholipids are commercially available reagents (Avanti Polar Lipids, Genzyme Pharmaceuticals, Lipoid), or can be prepared according to methods well known in the art. These PEG-lipid conjugates and their synthesis have been described in detail in Allen, T.M., Hansen, C., Martin, F., Redemann, C. and Yau-Young, A. 1991. Liposomes containing synthetic lipid derivatives of poly(ethylene glycol) show prolonged circulation half-lives in vivo. Biochim. Biophys. Acta 1066, 29-36, which is incorporated by reference in its entirety as if fully set forth. Phospholipids suitable for use in the invention may be any naturally occurring or synthetic phospholipid, whether saturated or unsaturated in the lipid portion of the molecule. They include, but are not limited to, the following: DSPE, dipalmitoylphosphatidylethanolamine (DPPE), phosphatidylcholine, phosphatidylethanolamine, phosphatidylserine, phosphatidylinositol, phosphatidylglycerol, phosphatidic acid, lysophospholipids, egg or soybean phospholipid or combinations thereof. The phospholipids may be in any form, including salted or desalted, hydrogenated or partially hydrogenated, or natural, semisynthetic (modified) or synthetic.

More preferred are saturated phosphatidylglycerols, phosphatidylethanolamines or phosphatidylcholines.

Without being bound by theory, and with respect to the rapid release characteristic of the micelles of the invention, it is believed that the combination of hydrophobic active agents with PEG containing phospholipids results in the hydrophobic active agent being transferred away from the amphipathic molecules when the formulation is diluted below the critical micelle concentration (CMC) of the mixture. Thus upon intravenous injection, the formulation undergoes a dilution in the blood which should be below the CMC of the formulation to ensure drug release.

Phosphatidylglycerols (PGs) may also be present in the micelles of the invention. Examples of such PGs include dimyristoyl phosphatidylglycerol (DMPG), DLPG and the like. Other types of suitable lipids that may be included are phosphatidylethanolamines (PEs), phosphatidic acids (PAs), phosphatidylserines, and phosphatidylinositols.

### Antioxidants

In embodiments comprising the use of unsaturated phospholipids, the invention may include the use of antioxidants to prevent oxidation of the phospholipids. Auto-oxidation of unsaturated acyl chains may be a problem for long-term storage of liposome formulations. Failure to prevent oxidative breakdown of unsaturated phospholipids results in subcomponents such as lyso lipids and fatty acids, which may be undesirable in some micelle compositions. As such, antioxidants suitable for inclusion in phospholipid containing micelles to improve long-term storage are known in the art. Examples of such antioxidants include butylated hydroxytoluene (BHT), alpha-tocopherol, and ascorbyl palmitate (AP) as well as pH buffering agents such as phosphates and glycine. Preferably, BHT is present at about 0.01-0.02% by weight and AP at about 0.1-0.2% by weight.

BHT is hydrophobic and would be expected to remain in the lipophilic environments of the micelles of the invention. BHT has the ability to prevent chain propagation during auto-oxidation by accepting radicals formed during the oxidative breakdown of lipids. Ascorbic acid has the capacity to act as an antioxidant and to act with other antioxidants such as alpha-tocopherol. It has been shown that the BHT/ascorbic acid system allows for BHT regeneration, following its conversion to a phenoxyl radical after free radical scavenging from oxidized lipids, thereby resulting in the appearance of ascorbyl radicals. This latter factor justifies the relative weight ration of AP to BHT described above. AP was used in place of ascorbic acid because the hydrophobic nature of the former would be expected to concentrate the antioxidant within lipophilic environments.

Another anti-oxidation consideration is the filling of container headspaces with nitrogen gas and the sealing of such containers. Additionally, and because metal ions can catalyze oxidative processes, the use of high quality drug, excipients, and containers, the judicious cleaning of manufacturing equipment, and the appropriate use of metal ion chelators are preferred.

### Cryoprotective Agents and Isotonic Agents

In a preferred embodiment of the invention, the micelles can be further stabilized by lyophilization. Micelles of the invention may contain a cryoprotectant for stabilization during lyophilization. Alternatively, the physical structures of the micelles can be preserved by the presence of sufficient water after lyophilization. This is may be accomplished by appropriate control of the degree of lyophilization.

Any cryoprotective agent known to be useful in the art of preparing freeze-dried formulations, such as di- or polysaccharides or other bulking agents such as lysine, may be used in the claimed invention. Further, isotonic agents typically added to maintain isomolarity with body fluids may be used. In preferred embodiments, a di-saccharide or polysaccharide is used and functions both as a cryoprotective agent and as an isotonic agent. In an especially preferred embodiment, the disaccharide or polysaccharide is selected from among the group consisting of lactose, trehalose, maltose, maltotriose, palatinose, lactulose or sucrose, with lactose or trehalose being preferred. Effective sugars such as trehalose and lactose are capable of hydrogen bonding to the phospholipidhead group in place of water.

The addition of a disaccharide or polysaccharide may be done during the manufacturing of the thin film, or alternatively, may be added after dry lipid film formation as a part of the aqueous solution used to hydrate the micelles.

Disaccharides or polysaccharides are preferred to monosaccharides for this purpose. To keep the osmotic pressure of the micelle compositions of the invention similar to that of blood, no more than 4-5% monosaccharides should be added. In contrast, about 9-10% of a disaccharide can be used without generating an unacceptable osmotic pressure. Also, when present, the disaccharide or polysaccharide is formulated in a preferred weight ratio of about 10-20 saccharide to 0.5-6.0 total phospholipids, respectively, even more preferably at a ratio from about 10 to 1.5-4.0. In one embodiment, a preferred but not limiting formulation is lactose or trehalose and total phospholipids in a ratio of about 10 to 0.94-1.88 to about 0.65-1.30, respectively.

### Freeze-drying

Once formulated, the micelles of the invention may be freeze-dried or lyophilized for long-term storage if desired. For example, BPD-MA, a preferred hydro-monobenzoporphyrin photosensitizer, has maintained its potency in a cryodesiccated composition for a period of at least nine months at room temperature, and a shelf life of at least two years has been projected. If the composition is freeze-dried, it may be packed in vials for subsequent reconstitution with a suitable aqueous solution, such as sterile water or sterile water containing a saccharide and/or other suitable excipients, just prior to use. For example, reconstitution may be by simply adding water for injection just prior to administration.

Various lyophilization techniques are known in the art. For example, micelle-containing vials of the invention may be first frozen to -45°C and then held there for a period of up to about 90 minutes. This may be followed by a high vacuum primary drying cycle wherein the temperature is increased slowly to up to about 10°C for a period usually on the order of about 50 hours. This may be followed by a 20°C secondary drying cycle of up to about 24 hours. Once the lyophilizer pressure stabilizes at about 55-65 mTorr (73-87 microbar), the cycle is terminated. Thereafter, the vials may be sealed after overlaying with nitrogen gas. A general rule for freeze-drying is that a solid, brittle, non-collapsed, and homogenous cake is preferred for successful re-hydration.

Additionally, the use of lyophilization may prevent hydrolysis of hydrophobic agents susceptible to such reactions. For example, the photosensitizer BPD-MA may be hydrolyzed to BPD-DA.

### Administration and Use

The use of the hydrophobic agents incorporated in the micelles of the invention may be for any appropriate pharmaceutical, agricultural or industrial application. With incorporated photosensitizers, the micelles may be used for any condition or in any method for which the photosensitizers are appropriate in combination with exposure to light or other electromagnetic radiation. These include, but are not limited to, the diagnosis or treatment of cancer, the reduction of levels of activated leukocytes, the treatment of ocular disorders, the treatment and prevention of neovasculature and angiogenesis, the destruction of viruses and cells infected thereby, the treatment of atherosclerotic plaques, the treatment of restenosis, and others. In addition, many photosensitizers may be photoactivated by appropriate excitation wavelengths to fluoresce visibly. This fluorescence can then be used to localize a tumor or other target tissue. By incorporating hydrophobic agents in the micelles of the invention, more efficient packaging, delivery and hence administration of the agents can be obtained.

Generally speaking, the micelles of the invention may be applied in any manner identical or analogous to the administration of micelles and liposomes. The concentration of the hydrophobic agent in the micelles of the invention depends upon the nature of the agent as well as the nature of the administration desired.

The micelle compositions and formulations of the invention may be administered parenterally or by injection. Injection may be intravenous, subcutaneous, intramuscular, intrathecal, intratumoral, or even intraperitoneal. However, the micelles may also be administered by aerosol intranasally or intrapulmonarally, or topically. Formulations designed for timed release are also with the scope of the invention.

The quantity of hydrophobic agent micelle formulation to be administered depends on the choice of active agents, the conditions to be treated, the mode of administration, the individual subject, as well as the skill, experience and judgement of the practitioner. Generally speaking, however, dosages in the range of 0.05-10 mg/kg may be appropriate. The foregoing range is, of course, merely suggestive, as the number of variables in regard to an individual treatment regime is large. Therefore, considerable excursions from these recommended values are expected. The quantity of photosensitive agent micelle formulation to administer *in vivo* can easily be determined by simple dose ranging studies that are well known in the art.

For example, and with the use of photosensitizers as a diagnostic in localizing tumor tissue or in localizing atherosclerotic plaques, the micelle compositions of the invention are administered systemically in the same general manner as is known with respect to photodynamic therapy. The waiting period to allow the drugs to clear from tissues to which they do not accumulate is approximately the same, for example, from about 30 minutes to about 10 hours. After the compositions of the invention have been permitted to localize, the location of the target tissue is determined by detecting the presence of the photosensitizer.

In diagnosis, the photosensitizers incorporated into micelles may be used along with, or may be labeled with, a radioisotope or other detecting means. If this is the case, the detection means depends on the nature of the label. Scintigraphic labels such as technetium or indium can be detected using *ex vivo* scanners. Specific fluorescent labels can also be used but, like detection based on fluorescence of the photosensitizers themselves, these labels may require prior irradiation.

For activation of the photosensitizer applied by the micelles of the invention, any suitable absorption wavelength is used. This can be supplied using the various methods known to the art for mediating cytotoxicity or fluorescence emission, such as visible radiation, including incandescent or fluorescent light sources or photodiodes such as light emitting diodes. Laser light can also be used for *in situ* delivery of light to a localized photosensitizer. In a typical protocol, for example, a few minutes to several hours prior to irradiation, approximately 0.5-1.5 mg/kg of green porphyrin photosensitizer containing micelle is injected intravenously and then excited by light of an appropriate wavelength-i.e. light containing a wavelength that is absorbed by the photosensitizer.

Preferably, electromagnetic radiation, such as from ultraviolet to visible and infrared light, is delivered after administration of the compositions and formulations of the invention. Generally, there are three significant variables - the concentration of the photosensitizing drug, the intensity of the radiation employed and the time of exposure to light, which determines the total amount of energy ultimately delivered to the target tissue. Generally, an increase in one of these factors permits a decrease in the others.

For example, if it is desired to irradiate only for a short period of time the energy of irradiation or the concentration of the drug may be increased. Conversely, if longer time periods of irradiation are permitted, lower irradiation intensities and lower drug concentrations are desirable. In some instances, the combination of 0.15 mg/kg body weight of BPD-MA as a drug dose and approximately 1-25 J/cm² total radiation from an appropriate radiation source provided successful results when low dose PDT is sufficient to produce the desired therapeutic effect. Low dose PDT is useful for such indications as treatment of autoimmune conditions, and prevention of inflammation. The use of low dose PDT offers an additional advantage in the form of reducing the likelihood of PDT side effects such as damage to unintended tissues. Higher doses of PDT are generally used for the destruction of target tissues such as tumor cells, as demonstrated in Example 4.

It is understood that the manipulation of these parameters will vary according to the nature of the tissue being treated and the nature of the photosensitizer (PS) employed. However, in general, low-dose PDT employs combinations of the drug concentration, radiation intensity, and total energy values which are several fold lower than those conventionally used for destroying target tissues such as tumors and unwanted neovascularization. One measure might be the product of PS concentration (e.g., in ng/ml) x intensity (e.g., in mW/cm2) x time (e.g., in seconds). However, it is difficult to set absolute numbers for this product since there are constraints on each of the parameters individually. For example, if the intensity is too low, the PS will not be activated consistently; if the intensity is too high, hyperthermic and other damaging effects may occur. Additionally, in some instances, ambient or environmental light available at the target cell or tissue undergoing PDT may be sufficient in the absence of additional deliberate irradiation.

Similarly, PS concentrations cannot vary over any arbitrary range. There may also be constraints on the time during which radiation can be administered. Accordingly, the product of the foregoing equation is only a rough measure. However, this approach may provide a convenient index that can be adjusted according to the relative potency of the PS employed, and in general, an increase in intensity would permit a decrease in time of irradiation, and so forth.

Having now generally described the invention, the same will be more readily understood through reference to the following examples, which are provided by way of illustration and are not intended to be limiting of the present invention, unless specified.

### EXAMPLE 1

### Preparation of Thin Films and Hydration

To make a 2 mg/mL active drug formulation, PEG₂₀₀₀-DSPE conjugate (obtained from Avanti Polar Lipids, 1 - 20 mg/mL) was dissolved in 15 mL of dichloromethane in a 100 mL round bottom flask. Molar lipid to drug ratio varied from 0.5:1 to 6:1. The PEG₂₀₀₀-DSPE dissolved very rapidly to a clear, colorless solution when swirled for a few minutes by hand. Five mg of QLT 0069 or verteporfin was then added to the round bottom flask followed with 10 - 25 mL of dichloromethane. The flask was swirled by hand for at least 5 minutes until no undissolved particulates were visible to the unaided eye. A thin film was generated by evaporation of dichloromethane using a rotary evaporator.

The following parameters were used to generate thin film:

| | |
|---|---|
| Water bath temperature: | 35°C |
| Flask rotation: | 35 rpm |
| Initial vacuum: | 600 mbar |

In general, the vacuum was increased in approximately 25 mbar increments. By 300 mbar, most of the dichloromethane was evaporated and the thin film was formed.

After the thin films were formed, all thin film-containing flasks were kept under high vacuum (10 mbar) for 1 hour. Depending on the pKa of the PS, the thin films were hydrated with various aqueous solutions such as 2.5 mL pH 8.5 20 mM phosphate buffer or 2.5 mL 9.2% (w/v) lactose as shown in Tables 1, 2, 3 and 4. Flasks were placed in an orbital shaker and shaken at 200 rpm for 40 minutes on average.

Alternatively, the solution may be aliquoted followed by lyophilization. Prior to hydration, the solid material may be warmed to room temperature while protected from light.

Hydrated micelles may be sterilized by passage through a 0.22 µm or smaller filter.

Sample results are shown in Tables 1,2,3, and 4. The A692/A720 ratio was used to determine the relative amount of QLT 0069 in monomer (A692) versus aggregated dimer (A720) form (Tables 1 and 2). The tendency of B-ring BPDs to self-associate into dimers is described in D. Delmarre et al, Can J. Chem. 79: 1069-1074 (2001).

**Table 1. Hydration of PEG₂₀₀₀-DSPE /QLT 0069 Micelles with Different pH Buffers**

| **Lipid:Drug** | **[Drug]** | **Hydration Solution** | **% Filtered** | **A692/720** |
|---|---|---|---|---|
| **(mol:mol)** | **(mg/mL)** | | **(0.22µ filter)** | |
| 5:1 | 1 | 5DW pH 4.0 | Filter blocked | 0.68 |
| | | | | |
| 5:1 | 1 | distilled water | 87.8 | 1.03 |
| | | | | |
| 5:1 | 1 | saline pH 5.5 | 95.5 | 1.02 |
| | | | | |
| 5:1 | 1 | phosphate buffer | 87.5 | 1.75 |
| | | 20 mM pH 5.5 | | |
| | | | | |
| 5:1 | 1 | phosphate buffer | 87.8 | 2.74 |
| | | 20 mM pH 7.0 | | |
| | | | | |
| 5:1 | 1 | phosphate buffer | 98.3 | 3.90 |
| | | 20 mM pH 7.5 | | |
| | | | | |
| 5:1 | 1 | phosphate buffer | 97.6 | 29.9 |
| | | 20 mM pH 8.0 | | |
| | | | | |
| 5:1 | 1 | phosphate buffer | 99.7 | 24.8 |
| | | 20 mM pH 8.5 | | |
| | | | | |
| 5:1 | 2 | phosphate buffer | 97.1 | 2.40 |
| | | 20 mM pH 7.5 | | |
| | | | | |
| 5:1 | 2 | phosphate buffer | 105.3 | 129 |
| | | 2 mM pH 7.5 | | |
| | | | | |
| 5:1 | 2 | distilled water | 91.0 | 0.85 |
| | | | | |
| 7:1 | 2 | phosphate buffer | 97.7 | 4.33 |
| | | 20 mM pH 7.5 | | |
| | | | | |
| 7:1 | 2 | distilled water | 90.7 | 2.04 |

**Table 2. Hydration of PEG₂₀₀₀-DSPE/QLT 0069 Micelles with pH 8.5 Phosphate Buffer**

| **Lipid:Drug** | **[Drug]** | **Hydration Solution** | **% Filtered** | **A692/A720** |
|---|---|---|---|---|
| **(mol:mol)** | **(mg/mL)** | | **(0.22µ filter)** | |
| 2:1 | 2.2 | phosphate buffer | 37.5 | 0.134 |
| | | 20 mM pH 8.5 | | |
| | | | | |
| 4:1 | 2.2 | phosphate buffer | 79.6 | 1.49 |
| | | 20 mM pH 8.5 | | |
| | | | | |
| 6:1 | 0.74 | phosphate buffer | 105.1 | 10.24 |
| | | 20 mM pH 8.5 | | |
| | | | | |
| 6:1 | 2.2 | phosphate buffer | 102.1 | 3.88 |
| | | 20 mM pH 8.5 | | |
| | | | | |
| 8:1 | 0.74 | phosphate buffer | 100.0 | 27.65 |
| | | 20 mM pH 8.5 | | |
| | | | | |
| 8:1 | 2.2 | phosphate buffer | 88.1 | 18.13 |
| | | 20 mM pH 8.5 | | |

Selected samples from Table 2 were tested for stability upon storage at room temperature for 24 or 48 hours or at 4°C for 24 or 48 hours or for 1 or 4 weeks. The samples were deemed stable by the lack of sediment or precipitation retained by 0.22 µm filtration.

Optionally, lactose, or another sugar, may be included in the hydrating solution or the combination of amphipathic molecules and active agent(s) to shorten hydration time.

Table 3 shows the results before and after filtration with verteporfin-containing micelles after hydration in 20 mM phosphate buffer at pH 8.5 (where "5DW" refers to 5% dextrose water). The micelles were fully solubilized, as demonstrated by their filterability through 0.22 micron filters.

**Table 3. Analysis of Hydrated Formulations (verteporfin, 2 mg/mL) + PEG₂₀₀₀-DSPE+ 20 mM Phosphate Buffer, pH 8.5) with Varying Lipid:Drug Ratios**

| | | | | | |
|---|---|---|---|---|---|
| **Lipid:Drug mol:mol Ratio** | 0.5:1 | 1:1 | 2:1 | 4:1 | 6:1 |

| **UV Analysis (mg/mL)** | | | | | |
|---|---|---|---|---|---|
| **(Unfiltered / 0.22 µm Filtered)** | | | | | |
| Stock Solution | 1.93 / 1.87 | 1.99 / 1.95 | 1.87 / 1.84 | 2.03 / 1.95 | 1.96 / 1.90 |
| Stock Solution | nd / 1.89 | | 1.88 / 1.84 | 1.94 / 1.98 | nd / 1.91 |
| Overnight 4°C | | nd / 1.78^{a} | | | |
| Diluted 1:20 in 5DW | 0.090 / 0.080 | 0.10 / 0.09 | 0.094 / 0.090 | 0.11 / 0.10 | 0.090 / 0.090 |
| Diluted 1:20 in 5DW | nd / 0.080 | | 0.091 /0.088 | 0.10 / 0.09 | nd / 0.092 |
| Overnight 4°C | | nd/ 0.08^{a} | | | |

| | | | | | |
|---|---|---|---|---|---|
| ^{a} These samples were stored for 3 days at 4°C, instead of just overnight. nd = not determined | | | | | |

Table 4 shows the results before and after filtration with micelles containing verteporfin after hydration in a solution containing 9.2% lactose.

**Table 4. Verteporfin (2 mg/mL) + PEG₂₀₀₀-DSPE Containing 9.2% Lactose Formulations (Unbuffered)**

| | | | |
|---|---|---|---|
| **Lipid:Drug mol:mol Ratio** | 0.5:1 | 2:1 | 6:1 |
| | | | |

| **UV Analysis (mg/mL)** | | | |
|---|---|---|---|
| **(Unfiltered / 0.22 µm Filtered)** | | | |
| Stock Solution | 2.12 / 1.53 | 2.15 / 2.08 | 2.01 / 1.97 |
| Stock Solution | nd | 2.15 / 2.12 | 2.01 / 2.00 |
| Overnight 4°C | | | |
| Diluted 1:20 in 5DW | 0.96 / 0.52 | 0.10 / 0.10 | 0.10 / 0.10 |
| Diluted 1:20 in 5DW | | 0.10 / 0.10 | 0.10 / 0.10 |
| Overnight 4°C | nd | | |

It should be noted that generally for QLT 0069 and other B-ring BPDs, about 6:1 (molar) lipid:photsensitizer ratio is needed to obtain a concentration of about 2 mg/mL of the photosensitizer while only about 2:1 is needed for A-ring BPDs such as verteporfin.

These results show that both the A-ring and B-ring benzoporphyrin derivatives can be readily entrapped in PEG₂₀₀₀-DSPE micelles at drug concentrations as high as 1-2 mg/ml. The incorporation efficiency of the B-ring benzoporphyrin derivative, QLT 0069 depended on both the lipid:drug molar ratio and the pH of the hydration medium used in the preparation. It should be noted that B-ring BPD micelles remained soluble (as determined by filterability through a 0.22 micron filter) after being rehydrated in aqueous media over a pH range of 5.5 to 8.5. However, there was a tendency of the drug to self-aggregate if the pH of the rehydration medium was not kept above the pKa of the carboxyl group in the molecule.

### EXAMPLE 2

### Critical micelle concentration (CMC) of PEG-phospholipid micelle with and without QLT 0069

Increasing the concentration of surfactant in aqueous solution causes a decrease in the surface tension of the solution until a certain concentration where it then becomes essentially constant with increasing concentration. The change occurs at the CMC. The most reasonable explanation of these effects is that surfactant molecule self-associates to form soluble aggregates, known as micelles. During the process of micelle formation, the hydrophobic groups form the core of the micelle and are shielded from the water to achieve a state of minimum free energy. The micelles are in dynamic equilibrium with free molecules (monomers) in solution; that is the micelles are continuously breaking down and reforming. Several physical properties change with increasing surfactant concentration above the CMC, e.g., surface tension, intensity of light scattering, osmotic pressure, equivalent conductivity, solubility of a water-insoluble solute. All of these properties could be used to determine the CMC of a surfactant. Here, we used light scattering to determine the CMC of P2K-DSPE (PEG₂₀₀₀-DSPE) in either distilled water or phosphate buffer.

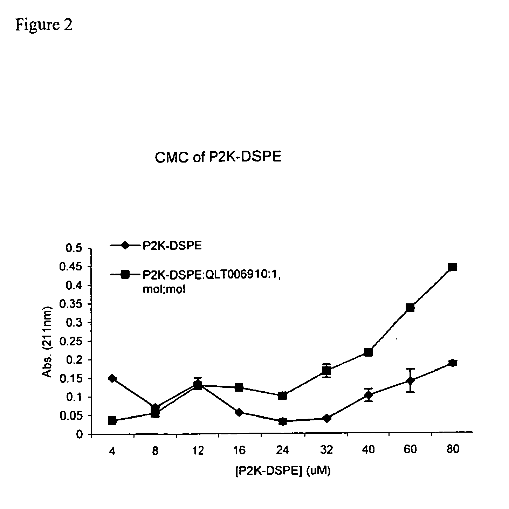

P2K-DSPE was initially dissolved in distilled water at concentrations between 4 - 80 µM. The absorption at 211 nm was read using a visible-UV spectrometer (Ultrospec® 3000, Pharmacia Biotech), and plotted as a function of P2K-DSPE concentration. The concentration at which the slope changes is related to the CMC. Results show that the absorption of P2K-DSPE increased at a concentration of 24 - 32 µM (Figure 2). This data is very close to the reported CMC (20 µM) from the previous studies in our lab. Figure 2 also shows the abrupt increase in the absorption of P2K-DSPE-micelles containing QLT0069. Thus, incorporation of QLT0069 into P2K-DSPE appears not to appreciably affect its CMC.

### EXAMPLE 3

### Effect of varying diacyl chain length and saturation; molecular weight of PEG

Studies were conducted to evaluate the incorporation of QLT0069, a B-ring BPD, into micelles composed of different PEG-lipid conjugates. These included:
- Poly(ethylene glycol)₂₀₀₀- dioleoylphosphatidylethanolamine (P2K-DOPE) (18:1) (Cat. 880130) obtained from Avanti Polar Lipids, Inc.
- Poly(ethylene glycol)₂₀₀₀- dipalmitoylphosphatidylethanolamine (P2K-DPPE) (16:0) (Cat. 880160) obtained from Avanti Polar Lipids, Inc.
- Poly(ethylene glycol)₅₀₀₀- dimyristoylphosphatidylethanolamine (P5K-DMPE) (14:0) (Cat. 880210) obtained from Avanti Polar Lipids, Inc.
- Poly(ethylene glycol)₅₀₀₀- dioleoylphosphatidylethanolamine (P5K-DOPE) (18:1) (Cat. 880230) obtained from Avanti Polar Lipids, Inc.
- Poly(ethylene glycol)₅₀₀₀- dipalmitoylphosphatidylethanolamine (P5K-DPPE) (16:0) (Cat. 880200) obtained from Avanti Polar Lipids, Inc.
- Poly(ethylene glycol)₅₀₀₀- distearoylphosphatidylethanolamine (P5K-DSPE) (18:0) (Cat. 880220) obtained from Avanti Polar Lipids, Inc.
- Poly(ethylene glycol)₇₅₀- distearoylphosphatidylethanolamine (PEG₇₅₀-DSPE) (18:0) (Cat. 880620) obtained from Avanti Polar Lipids, Inc.
- Poly(ethylene glycol)₁₇₅₀-steric acid (PEG₁₇₅₀-Steric acid)
Micelles were prepared following the procedure outlined in Example 1, except that in some of the samples, various other PEG-lipid conjugates were substituted for P2K-DSPE. The drug concentrations varied from 0.02 to 0.2 mg/ml. The thin film samples were all hydrated in an aqueous buffer at physiological pH and osmolarity using 20 mM MOPS, 5% Dextrose USP, pH. 7.0. The efficacy of the various micelle formulations in preventing self-aggregation of the PS BPD-MB (measured as A₆₉₂/A₇₂₀) is shown in Table 5. The presence of either dimer (A₇₂₀) or monomer (A₆₉₂) forms of BPD-MB in micelles was related to the degree of fatty acyl chain saturation and chain length. For example, at the lipid:drug molar ratio of 5:1, the drug incorporated into either P5K-DSPE or P5K-DPPE micelles was present as monomers, while drug dimers were present in micelles formed from either P5K-DMPE or P5K-DOPE. Drug formulated into PEG₁₇₅₀-steric acid micelles hydrated very rapidly although the drug was present mainly as dimers.

When loading QLT0069 into PEG-lipid micelles, the most homogeneous monomer-containing formulation was obtained when long, saturated, acyl chains were used in the micelle hydrophobic core. PEG-DSPE, with PEG molecular weights of 2000 or 5000, formed micelles with equal efficiency. Since P2K-DSPE has already been approved as an excipient in liposomal formulations (Doxil®, Alza Corporation), it is a good candidate for micelles intended for clinical use.

**Table 5. Determination of the optimal mPEG-lipid for the loading of QLT0069**

| **mPEG-lipids** | **mPEG-lipid:drug ratios** | **monomer/dimer Ratios** |
|---|---|---|
| | **(mol:mol)** | **(A₆₉₂ /A₇₂₀)** |
| P5K-DSPE | 5 : 1 | 47 |
| | 10 : 1 | 57 |
| | 20 : 1 | 50 |
| P5K-DPPE | 5 : 1 | 15 |
| | 10 : 1 | 38 |
| | 20 : 1 | 62 |
| P5K-DMPE | 5 : 1 | 2.1 |
| | 10 : 1 | 68 |
| | 20 : 1 | 53 |
| P5K-DOPE | 5 : 1 | 1.7 |
| | 10 : 1 | 34 |
| | 20 : 1 | 54 |
| P2K-DSPE | 5 : 1 | 36 |
| | 10 : 1 | 63 |
| | 20 : 1 | 56 |
| P2K-DOPE | 5 : 1 | 1 |
| | 10 : 1 | 10 |
| | 20 : 1 | 67 |
| PEG₁₇₅₀-steric acid | 5 : 1 | 0.4 |
| | 10 : 1 | 0.9 |
| | 20 : 1 | 2.6 |

| | | |
|---|---|---|
| Monomer:dimer ratios were determined for samples diluted in the hydration buffer to a final concentration of 10-30 µM. | | |

### EXAMPLE 4

### In vivo use of micelles

The ability of PDT with PEG2000-DSPE formulations of photosensitizer to control tumor growth was assessed in DBA 2 mice using an M1 tumor model. Male mice were implanted with 2 X 10⁴ M1 tumor cells in a 50 µl volume, and maintained until the tumor had grown to 4-6 mm in diameter. Either a single intravenous dose of 1.4 µmol/kg or a single intratumoral injection of 0.09 mg of PS (active ingredient) was administered to DBA mice. After a waiting period ranging from 15 to 60 minutes, 50J/cm² of 690 nm light was administered to the tumor site at a fluence rate of 90 mW/cm². The results are shown in Table 6. QLT0069-PEG ₂₀₀₀-DSPE was as effective as the liposomally-formulated A-ring BPD, QLT0074, in controlling tumors when delivered intratumorally. Verteporfin-PEG₂₀₀₀-DSPE was highly effective in tumor control when administered intravenously.

**Table 6. Results of Tumor Bioassay - the Number of Tumor-free Animals**

| **Drug** | **Time of Irradiatio** | **# of Tumor Free Mice** | | | |
|---|---|---|---|---|---|
| **(Formulation)** | | **Day 3** | **Day 7** | **Day 14** | **Day 20** |
| QLT0069^{a} - IV | 30 min | 0/3^{b} | 0/3 | 0/3 | 0/3 |
| 6:1 PEG₂₀₀₀-DSPE: drug | 60 min | 0/3 | 0/3 | 0/3 | 0/3 |
| | 180 min | 0/3 | 0/3 | 0/3 | 0/3 |
| | | | | | |
| Verteporfin ^{a}- IV | 15 min | 3/3 | 3/3 | 3/3 | 3/3 |
| 6:1 PEG₂₀₀₀-DSPE: drug | 30 min | 3/3 | 3/3 | 3/3 | 3/3 |
| | 60 min | 3/3 | 3/3 | 3/3 | 2/3 |
| | | | | | |
| Verteporfin ^{a}- IV | 15 min | 3/3 | 3/3 | 3/3 | 3/3 |
| 2:1 PEG₂₀₀₀-DSPE: drug | 30 min | 3/3 | 3/3 | 3/3 | 2/3 |
| | 60 min | 3/3 | 3/3 | 2/3 | 1/3 |
| | | | | | |
| QLT0069 ^{b} - intratumoral | 30 min | 3/3 | 3/3 | 0/3 | 0/3 |
| 6:1 PEG₂₀₀₀-DSPE: drug | 45 min | 3/3 | 3/3 | 0/3 | 0/3 |
| | 60 min | 3/3 | 2/3 | 0/3 | 0/3 |
| | | | | | |
| QLT0074 ^{b} - intratumoral | 30 min | 3/3 | 3/3 | 0/3 | 0/3 |
| (liposomal formulation) | 45 min | 3/3 | 3/3 | 0/3 | 0/3 |
| | 60 min | 3/3 | 2/3 | 0/3 | 0/3 |

| | | | | | |
|---|---|---|---|---|---|
| ^{a} M1 tumors in DBA/2 mice were treated with PDT: (50 J/cm² of 690 nm light at 30 - 60 min following IV administration of QLT0069 or verteporfin at 1.4 µmol/kg. ^{b} M1 tumors in DBA/2 mice were treated with PDT (50 J/cm² of 690 nm light at 30 - 60 min following single intra-tumoral injection of 0.094 mg (QLT0069 or QLT0074) | | | | | |

### EXAMPLE 5

### Particularly preferred embodiments

A particularly preferred embodiment of the invention is a micelle-containing composition comprising one or more photosensitizer and one or more than one PEG-containing phospholipids which form micelles. Such compositions preferably comprise a green porphyrin photosensitizer which is preferably vertepofrinor QLT0069. The particularly preferred compositions may also comprise PEG-2000, at least distearoylphosphatidylethanolamine (DSPE) is said one or more than one phospholipid, or PEG₂₀₀₀-DSPE as said one or more than one phospholipid.

The particularly preferred compositions preferably comprise a molar ratio of lipid : photosensitizer of between about 0.5 and about 10. More preferably, the composition comprises QLT 0069 and PEG₂₀₀₀-DSPE, wherein the lipid : photosensitizer molar ratio ranges between about 6 and about 10. Also preferred is a composition comprising verteporfin and PEG₂₀₀₀-DSPE, wherein the lipid : photosensitizer molar ratio ranges between about 1 and about 6. In any particularly preferred compositions, the photosensitizer concentration is about 1-2 mg/ml and/or lyophilized.

### REFERENCES

1. Sou K, Endo T, Takeoka S, Tsuchida E. Poly(ethylene glycol)-modification of the phospholipid vesicles by using the spontaneous incorporation of poly(ethylene glycol)-lipid into the vesicles. Bioconjug Chem. 2000;11(3):372-379.
2. Lasic DD. Liposomes: from Physics to Applications. New York, NY: Elsevier. 1993.
3. Perkins WR, Ahmad I, Li X, et al. Novel therapeutic nano-particles (lipocores): trapping poorly water soluble compounds. Int J Pharm. 2000;200(1):27-39.
4. Trubetskoy VS, Torchilin VP. Use of polyoxyethylene-lipid conjugates as long-circulating carriers for delivery of therapeutic and diagnostic agents. Adv Drug Deliv Rev. 1995;16:311-320.
5. Onyuksel H, Ikezaki H, Patel M, Gao XP, Rubinstein I. A Novel Formulation of VIP in Sterically Stabilized Micelles Amplifies Vasodilation In Vivo. Pharm Res. 1999;16(1):155-160.
6. Gabizon A, Isacson R, Libson E, et al. Clinical studies of liposome-encapsulated doxorubicin. Acta Oncol. 1994;33(7):779-786.
7. Aveline B, Hasan T, Redmond R. Photophysical and photosensitizing properties of benzoporphyrin derivative monoacid ring A (BPD-MA). Photochem Photobiol. 1994;59(3):328-335.

## Claims

1. A micelle-containing composition comprising
a) one or more photosensitizer and
b) one or more PEG-containing phospholipids which form micelles.

2. The composition of claim 1 wherein said photosensitizer is a green porphyrin.

3. The composition of claim 2 wherein the green porphyrin is verteporfin.

4. The composition of claim 2 wherein the green porphyrin is QLT 0069.

5. The composition of any one of claims 1-4 which comprises PEG₂₀₀₀₋

6. The composition of any one of claims 1-5 further comprising a phospholipid that is distearoylphosphatidylethanolamine (DSPE).

7. The composition of any one of claims 1-6 in which a PEG-containing phospholipid is PEG₂₀₀₀-DSPE.

8. The composition of any one of claims 1-7 in which the molar ratio of lipid : photosensitizer is between about 0.5 and about 10.

9. A micelle-containing composition comprising a green porphyrin photosensitizer, and one or more PEG-containing phospholipid that form micelles.

10. A micelle-containing composition comprising QLT 0069 photosensitizer and PEG₂₀₀₀-DSPE, wherein the lipid : photosensitizer molar ratio ranges between about 6 and about 10.

11. A micelle-containing composition comprising veteporfin photosensitizer and PEG₂₀₀₀-DSPE, wherein the lipid: photosensitizer molar ratio ranges between about 2 and about 6.

12. The composition of claim 10 or claim 11 wherein the photosensitizer concentration is about 2 mg/ml.

13. The composition of any one of claims 1-12 in a lyophilized form.

## Patentansprüche

1. Mizellenhältige Zusammensetzung, umfassend
a) einen oder mehrere Photosensitizer, sowie
b) ein oder mehrere PEG-hältige Phospholipide, die Mizellen bilden.

2. Zusammensetzung nach Anspruch 1, worin der Photosensitizer ein grünes Porphyrin ist.

3. Zusammensetzung nach Anspruch 2, worin das grüne Porphyrin Verteporfin ist.

4. Zusammensetzung nach Anspruch 2, worin das grüne Porphyrin QLT 0069 ist.

5. Zusammensetzung nach einem der Ansprüche 1 bis 4, die PEG₂₀₀₀ umfasst.

6. Zusammensetzung nach einem der Ansprüche 1 bis 5, die weiters ein Phospholipid umfasst, das Distearoylphosphatidylethanolamin (DSPE) ist.

7. Zusammensetzung nach einem der Ansprüche 1 bis 6, worin ein PEG-hältiges Phospholipid PEG₂₀₀₀-DSPE ist.

8. Zusammensetzung nach einem der Ansprüche 1 bis 7, worin das Molverhältnis Lipid:Photosensitizer zwischen etwa 0,5 und etwa 10 beträgt.

9. Mizellenhältige Zusammensetzung, die einen grünen Porphyrin-Photosensitizer und ein oder mehrere PEG-hältige Phospholipide enthält, die Mizellen bilden.

10. Mizellenhältige Zusammensetzung, die QLT-0069 als Photosensitizer und PEG₂₀₀₀-DSPE enthält, worin das Molverhältnis Lipid:Photosensitizer zwischen etwa 6 und etwa 10 beträgt.

11. Mizellenhältige Zusammensetzung, die Verteporfin als Photosensitizer und PEG₂₀₀₀-DSPE enthält, worin das Molverhältnis Lipid:Photosensitizer zwischen etwa 2 und etwa 6 beträgt.

12. Zusammensetzung nach Anspruch 10 oder Anspruch 11, worin die Photosensitizer-Konzentration etwa 2 mg/ml beträgt.

13. Zusammensetzung nach einem der Ansprüche 1 bis 12 in lyophilisierter Form.

## Revendications

1. Composition contenant des micelles comprenant :
a) un ou plusieurs photosensibilisateurs, et
b) un ou plusieurs phospholipides contenant du PEG qui forment des micelles.

2. Composition selon la revendication 1, dans laquelle ledit photosensibilisateur est une porphyrine verte.

3. Composition selon la revendication 2, dans laquelle la porphyrine verte est la vertéporfine.

4. Composition selon la revendication 2, dans laquelle la porphyrine verte est le QLT 0069.

5. Composition selon l'une quelconque des revendications 1 à 4, qui comprend le PEG₂₀₀₀.

6. Composition selon l'une quelconque des revendications 1 à 5, comprenant en outre un phospholipide qui est la distéaroylphosphatidyléthanolamine (DSPE).

7. Composition selon l'une quelconque des revendications 1 à 6, dans laquelle un phospholipide contenant du PEG est la DSPE-PEG₂₀₀₀.

8. Composition selon l'une quelconque des revendications 1 à 7, dans laquelle le rapport molaire de lipide : photosensibilisateur est compris entre environ 0,5 et environ 10.

9. Composition contenant des micelles, comprenant un photosensibilisateur de porphyrine verte, et un ou plusieurs phospholipides contenant du PEG qui forment des micelles.

10. Composition contenant des micelles, comprenant un photosensibilisateur de QLT 0069 et la DSPE-PEG₂₀₀₀, dans laquelle le rapport molaire lipide : photosensibilisateur est compris entre environ 6 et environ 10.

11. Composition contenant des micelles, comprenant un photosensibilisateur de vertéporfine et la DSPE-PEG₂₀₀₀, dans laquelle le rapport molaire lipide : photosensibilisateur est compris entre environ 2 et environ 6.

12. Composition selon la revendication 10 ou la revendication 11, dans laquelle la concentration en photosensibilisateur est d'environ 2 mg/mL.

13. Composition selon l'une quelconque des revendications 1 à 12, sous une forme lyophilisée.
